# EUROPEAN PATENT APPLICATION

(11) **EP 4 260 833 A1**
(43) Date of publication of application: **18.10.2023**
(21) Application number: 21902285.2
(22) Date of filing: 04.11.2021
(51) Int. Cl.: A61F 2/24

(54) **PROSTHETIC HEART VALVE**

(30) Priority: 11.12.2020 CN 202011459644
(71) Applicant: Shanghai MicroPort CardioFlow Medtech Co., Ltd., Shanghai 201203 (CN)
(72) Inventor: JIN, Liang, Shanghai 201203 (CN); LIU, Shihong, Shanghai 201203 (CN); QIU, Yao, Shanghai 201203 (CN); JI, Lijun, Shanghai 201203 (CN); CHEN, Guoming, Shanghai 201203 (CN)
(74) Representative: Patentship Patentanwaltsgesellschaft mbH
(86) International application number: PCT/CN2021/128826
(87) International publication number: WO 2022/121578

(57) **Abstract**

An artificial heart valve prosthesis, including: a stent (1000) comprising at least two commissures (1100) spaced circumferentially around the stent, the commissure (1100) provided with a first window (1110) and a second window (1120), which are arranged along an axial direction of the stent (1000), the first window (1110) located closer to an outflow end (1002) of the stent (1000); and at least two leaflets (3100) arranged circumferentially around the stent (1000), each of the leaflets (3100) including a main segment (3110) and flaps (3120) arranged on opposite circumferential sides of the main segment (3110), the main segment (3110) disposed on an inner side of the stent (1000), the flap (3120) passed through the second window (1120) and covering at least a part of an outer surface of the commissure (1100), the flap (3120) sutured at the first window (1110) and the second window (1120). Through suturing the flaps (3120) of the leaflets (3100) at the first windows (1110), stress can be distributed across the joints of the leaflets (3100) with the stent (1000), resulting in an extended life of service of the leaflets (3100) and avoiding the leaflets (3100) from sagging during use of the artificial heart valve.

## Description

### TECHNICAL FIELD

The present invention relates to the field of medical devices and, in particular, to artificial heart valve prostheses.

### BACKGROUND

Heart valves include the aortic valve that connects the left ventricle to the aorta, the pulmonary valve that connects the right ventricle to the pulmonary artery, the mitral valve that connects the left atrium to the left ventricle and the tricuspid valve that connects the right atrium to the right ventricle. All these heart valves function like one-way valves and rhythmically open and close with the rhythmic contraction and dilation of the heart in the blood circulation, allowing smooth passage of blood through heart valves in one direction and preventing its passage in the opposite direction, hence to enable the blood to circulate in a certain direction within a body. Inflammation of heart valves may cause structural damage, fibrosis, adhesions, shortening, myxomatous lesions, ischemic necrosis, calcium precipitation and other problems affecting normal blood circulation, which are collectively called heart valve disease.

An artificial heart valve prosthesis is a device that can be implanted into the heart to function in place of the native heart valve. For severe valvular disease that cannot be recovered or functionally improved by valvuloplasty or valvular repair, it is necessary to replace the diseased native valve with an implanted artificial heart valve prosthesis. Such an artificial heart valve prosthesis includes a stent and a valve attached to an inner surface of the stent. In practical applications, limited by the material of the valve and other factors, commissural joints of the valve with the stent tend to be overstressed and calcified.

### SUMMARY OF THE INVENTION

It is an object of the present invention to provide an artificial heart valve prosthesis, which has an extended life thanks to its ability of effective stress distribution within joints of a valve structure with a stent.

To this end, the present invention provides an artificial heart valve prosthesis, comprising:
a stent comprising at least two commissures spaced circumferentially around the stent, each of the commissures provided with a first window and a second window, which are arranged along an axial direction of the stent, the first window located closer to an outflow end of the stent; and
at least two leaflets arranged circumferentially around the stent, each of the leaflets comprising a main segment and flaps arranged on opposite circumferential sides of the main segment, the main segment disposed on an inner side of the stent, the flap passed through the second window and at least partially covering an outer surface of the commissure, the flap sutured at the first window and the second window.

Optionally, the artificial heart valve prosthesis may further comprise hinge sheets, which are at least partially arranged between the commissure and the flaps of the leaflet, wherein the hinge sheet is provided with a through hole for passage therethrough of the flap and wherein the hinge sheet is sutured at the first window.

Optionally, the hinge sheet may cover the first window. Alternatively, the flaps may be sutured to the hinge sheet.

Optionally, the second window may be delimited by side support arms arranged circumferentially around the stent, wherein:
the hinge sheet at least partially covers an outer surface, an outer side face and an inner surface of the side support arm; and
each of the flaps comprises a head portion, a middle portion and a trailing portion, which are sequentially joined, the head portion inserted in the second window and the through hole, the middle portion extending over the outer surface, the outer side face and the inner surface of the side support arm so as to wrap a portion of the hinge sheet arranged on the side support arm, the trailing portion overlapping a portion of the main segment in the leaflet.

Optionally, the flap may be sutured at the second window by a suture thread successively passed through the main segment of the leaflet, the second window, the through hole and the trailing portions of the flap.

Optionally, in the circumferential direction of the stent, edges of the through hole may be coincident with or lateral to edges of the second window.

Optionally, the flap may be provided with an engagement notch on a side thereof proximate an inflow end of the stent, which are located at a junction of the flap with the main segment and configured to engage the through hole and the second window.

Optionally, the commissure may be further provided with a third window, which are located on a side of the second window proximate an inflow end of the stent,
wherein the hinge sheet is sutured at the third window.

Optionally, the second window may be delimited by side support arms arranged circumferentially around the stent,
wherein each of the flaps comprises a head portion, a middle portion and a trailing portion, which are sequentially joined, the head portion inserted in the through hole, the middle portion extending over outer surface, outer side face and inner surface of the side support arms, the trailing portion overlapping a portion of the main segment in the leaflet.

Optionally, the flap may be sutured at the second window by a suture thread successively passed through the main segment of the leaflet, the second window and the trailing portion of the flap.

To the above end, the present invention also provides another artificial heart valve prosthesis, comprising:
a stent comprising at least two commissures spaced circumferentially around the stent, the commissure provided with a second window;
hinge sheets at least partially covering an outer surface of the commissure and provided with a through hole; and
at least two leaflets arranged circumferentially around the stent, each of the leaflets comprising a main segment and flaps arranged on opposite circumferential sides of the main segment, the main segment disposed on an inner side of the stent, the flap passed through the second window and the through hole and covering at least a part of an outer surface of the hinge sheet, the flap sutured at the second window.

Optionally, the second windows may be delimited by side support arms arranged circumferentially around the stent, wherein:
the hinge sheets at least partially covers an outer surface, an outer side face and an inner surface of the side support arm; and
each of the flaps comprises a head portion, a middle portion and a trailing portion, which are sequentially joined, the head portion inserted in the second window and the through hole, the middle portion extending over the outer surface, the outer side face and the inner surface of the side support arms so as to wrap a portion of the hinge sheet arranged on the side support arm, the trailing portion overlapping a portion of the main segment in the leaflet.

Optionally, the flap may be sutured at the second window by a suture thread successively passed through the main segment of the leaflet, the second window, the through hole and the trailing portion of the flap.

Optionally, in the circumferential direction of the stent, edges of the through holes may be coincident with or lateral to edges of the second window.

Optionally, the flap may be provided with an engagement notch on a side thereof proximate an inflow end of the stent, which are located at a junction of the flap with the main segment and configured to engage the through hole and the second window.

Optionally, the commissure may be further provided with a third window, which are located on a side of the second window proximate an inflow end of the stent,
wherein the hinge sheet is sutured at the third window.

Compared with the prior art, the artificial heart valve prostheses of the present invention have the advantages as follows:
First, the artificial heart valve prosthesis includes a stent and at least two leaflets. The stent includes at least two commissures spaced circumferentially around the stent. The commissure is provided with a first window and a second window, which are arranged along an axial direction of the stent. The first window is located closer to an outflow end of the stent. The at least two leaflets are arranged circumferentially around the stent. Each of the leaflets includes a main segment and flaps arranged on opposite circumferential sides of the main segment. The main segment is disposed on an inner side of the stent, and the flap is passed through the second window and at least partially covers an outer surface of the commissure. The flap is sutured at the first window and the second window. In addition to being sutured at the second window, the flap is also sutured at the first window. In this way, stress is distributed across the joints of the leaflet with the stent (i.e., areas around the second window), resulting in an extended life of service of the leaflets. Moreover, since the first window is closer to the outflow end of the stent, the leaflet can be prevented from sagging during use of the artificial heart valve prosthesis.

Second, the artificial heart valve prosthesis may further include hinge sheet, which is at least partially arranged between the commissure and the flap to reduce friction between the flap and the stent. In particular, the second window may be delimited by side support arms arranged circumferentially around the stent. Moreover, the hinge sheet may at least partially cover an outer surface of the side support arms and completely covers outer side face and inner surface of the side support arm. Additionally, each of the flaps may include a head portion, a middle portion and a trailing portion, which are sequentially joined. The head portion may be inserted in the second window and the through hole, and the middle portion may extend over the outer surface, the outer side face and the inner surface of the side support arm so as to wrap a portion of the hinge sheet arranged on the side support arm. The trailing portions may overlap a portion of the main segment in the leaflet. With this arrangement, stress can be uniformly distributed across the entire commissure, thus effectively preventing stress concentration and improving the stent's durability.

Third, the flap may be overseamed with a suture thread that is successively passed through the main segment of the leaflet, the second window, the through hole and the trailing portion and then again through the main segment in such a manner the suture thread do not come into contact with the side support arms. In this way, the risk of the suture thread breaking due to rubbing against the side support arms is eliminated.

Fourth, the other artificial heart valve prosthesis includes a stent, hinge sheets and at least two leaflets arranged circumferentially around the stent. The stent includes at least two commissures, which are spaced circumferentially around the stent and provided with second windows. The hinge sheet at least partially covers an outer surface of the commissure and is provided with a through hole. Each of the leaflets includes a main segment and flaps arranged on opposite circumferential sides of the main segment. The main bodies are disposed on an inner side of the stent, and the flap is passed through the second window and the through hole and at least partially covers an outer surface of the hinge sheet. Moreover, the flaps are sutured at the second windows. Through arranging the hinge sheet between the commissure and the flap of the leaflet, friction between the commissure and the flap can be effectively reduced, resulting in an extended life of service of the leaflet.

### BRIEF DESCRIPTION OF THE DRAWINGS

The accompanying drawings are provided to facilitate a better understanding of the present invention and do not limit the scope thereof in any sense, in which:
Fig. 1 is a schematic diagram showing the structure of an artificial heart valve prosthesis according to an embodiment of the present invention;
Fig. 2 is a partial cross-sectional view of an artificial heart valve prosthesis according to an embodiment of the present invention;
Fig. 3 is a schematic unrolled plan view of a stent in an artificial heart valve prosthesis according to an embodiment of the present invention;
Fig. 4 is an enlarged schematic view of an commissure of the stent in the artificial heart valve prosthesis of Fig. 3;
Fig. 5 is a schematic diagram showing the structure of a hinge sheet in an artificial heart valve prosthesis according to an embodiment of the present invention;
Fig. 6 is a schematic diagram showing the structure of a leaflet in an artificial heart valve prosthesis according to an embodiment of the present invention;
Fig. 7 schematically illustrates how leaflets are hinged to a hinge sheet in an artificial heart valve prosthesis according to an embodiment of the present invention;
Fig. 8 is a cross-sectional view of the leaflets and the hinge sheet in the artificial heart valve prosthesis of Fig. 7, which are being hinged together;
Fig. 9 schematically illustrates assembly of leaflets and hinge sheets with a stent in an artificial heart valve prosthesis according to an embodiment of the present invention;
Fig. 10 is a schematic diagram showing the structure of a skirt in an artificial heart valve prosthesis according to an embodiment of the present invention.

### DETAILED DESCRIPTION

Particular embodiments of the present invention will be described below by way of specific examples. Based on the disclosure and teachings provided herein, a person of ordinary skill in the art will readily realize other advantages and benefits provided by the present invention. The present invention may also be otherwise embodied or applied through different embodiments, and various modifications or changes may be made to the details disclosed herein from different points of view or for different applications, without departing from the spirit of the present invention. It should be noted that the accompanying drawings are provided herein merely to schematically illustrate the basic concept of the present invention. Accordingly, they only show components relating to the present invention but not necessarily depict all the components as well as their real shapes and dimensions in practical implementations. In practice, the configurations, counts and relative scales of the components may vary arbitrarily and their arrangements may be more complicated.

In the following, each of the embodiments is described as having one or more technical features. However, this does not mean that the present invention must be practiced necessarily with all such technical features, or separately with some or all the technical features in any of the embodiments. In other words, as long as the present invention can be put into practice, a person skilled in the art may choose some or all of the technical features in any of the embodiments or combine some or all of the technical features in different embodiments based on the teachings herein and depending on relevant design specifications or the requirements of practical applications. In this way, the present invention can be carried out more flexibly.

As used herein, the singular forms "a", "an" and "the" include plural referents, and the plural form "a plurality of' means "two or more", unless the context clearly dictates otherwise. As used herein, the term "or" is generally employed in the sense including "and/or", unless the context clearly dictates otherwise. The terms "mounting", "coupling" and "connection" should be interpreted in a broad sense. For example, a connection may be a permanent, detachable or integral connection, or a mechanical or electrical connection, or a direct or indirect connection with one or more intervening media, or an internal communication or interaction between two elements. Those of ordinary skill in the art can understand the specific meanings of the above-mentioned terms herein, depending on their context.

As used herein to describe an artificial heart valve prosthesis, the term "inner" refers to a direction toward a central axis of the prosthesis, and the term "outer" refers to a direction away from the central axis.

Objects, advantages and features of the present invention will become more apparent upon reading the following more detailed description of the present invention, which is made with reference to the accompanying drawings. Note that the figures are provided in a very simplified form not necessarily drawn to exact scale and for the only purpose of facilitating easy and clear description of the embodiments. Throughout the annexed figures, like numerals indicate like elements.

Any valve mentioned in the following description refers to an artificial heart valve, unless it is stated as a native valve.

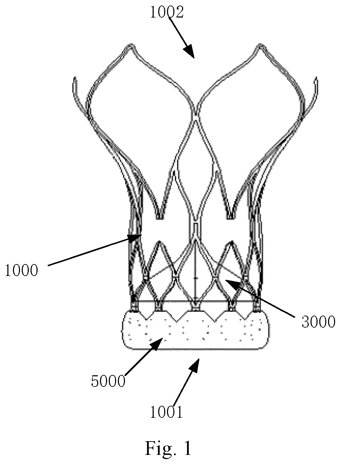

Referring to Figs. 1 to 6, an artificial heart valve prosthesis provided in a preferred embodiment of the present invention includes a stent 1000, hinge sheets 2000 and a valve 3000. Both the hinge sheets 2000 and the valve 3000 are attached to the stent 1000, and the valve 3000 includes at least two leaflets 3100.

With particular reference to Figs. 1, 3 and 4, the stent 1000 is a ring-shaped mesh structure, which is constructed from multiple struts and comprises an inflow end 1001 and an outflow end 1002. Here, the inflow end 1001 refers to an end for passage of blood therethrough into the stent, and the outflow end 1002 refers to an end for passage of blood therethrough out of the stent. The ring-shaped structure may be either regular, such as a circle, rectangle or regular polygon, or irregular. For ease of understanding, the ring-shaped structure is described in this embodiment as a circular structure as an example, but this should not be construed as limiting the present invention in any sense. The stent 1000 has at least two commissures 1100 spaced circumferentially around the stent. In each of the commissures 1100, a first window 1110 and a second window 1120 are formed along an axial direction of the stent 1000. The first window 1110 is closer to the outflow end 1002. That is, the second window 1120 and the first window 1110 are sequentially arranged in the direction from the inflow end 1001 to the outflow end 1002 of the stent 1000. In other words, here, the term "axial direction" refers to a direction parallel to the direction of blood flow after the artificial heart valve prosthesis is implanted into the heart.

Optionally, the first window 1110 may be a circular opening, and the second window 1120 may be a rectangular opening extending along the axial direction of the stent 1000. In other implementations, the first window 1110 may be otherwise shaped, such as a rectangle. Alternatively, the second window 1120 may have another elongate shape, such as oblong.

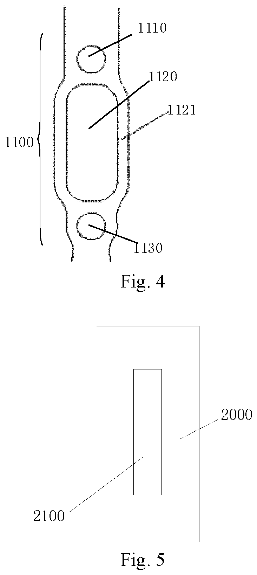

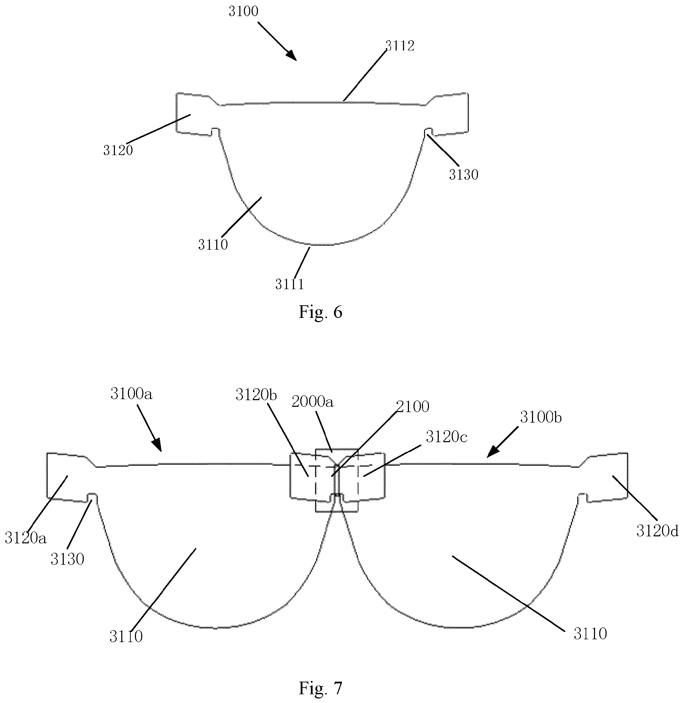

Referring to Fig. 5, each hinge sheet 2000 covers at least a part of an outer surface of a respective one of the commissures 1100 and comprises a through hole 2100. With further reference to Fig. 6, the at least two leaflets 3100 are arranged circumferentially around the stent 1000. Each of the leaflets 3100 includes a main segment 3110 and flaps 3120 arranged on opposite circumferential sides of the main segment 3110. The main segment 3110 is disposed on an inner side of the stent 1000, with each of the flaps 3120 passing through the respective second window 1120 and the respective through hole 2100 from the inner side of the stent 1000 and covering at least a part of the outer surface of the respective hinge sheet 2000. The flap 3120 is then sutured at the respective first window 1110 and the respective second window 1120. Suturing the flap 3120 at the first window 1110 can prevent the valve 3000 from sagging during use. Preferably, each hinge sheet 2000 is also sutured at the respective first window 1110, thereby achieving stress distribution while additionally preventing the valve 3000 from sagging during use.

It would be appreciated that the outer surface of the hinge sheet 2000 is a surface thereof facing away from the respective commissure 1100. Moreover, each hinge sheet 2000 sutured at the first window 1110 means that a suture thread is passed through the first window 1110 so as to suture the hinge sheet 2000 to the commissure 1100. Similarly, each flap 3120 sutured at the first window 1110 means that a suture thread passed through the first window 1110 so as to suture the flap 3120 to the commissure 1100. Additionally, each flap 3120 sutured at the second window 1120 means that a suture thread is passed through the second window 1120 so as to suture the flap 3120 to the commissure 1100. Further, in this embodiment, the flaps 3120 may be additionally sutured to the hinge sheet 2000 to facilitate relative positioning of them so that they stay at stable relative positions during the subsequent suturing of them to the commissures 1100. Furthermore, in order to enable successful passage of the flaps 3120 through the second windows 1120 and the through holes 2100, the through holes 2100 are preferably elongate hole along the axial direction of the stent 1000, and the through holes 2100 and the second windows 1120 are properly arranged so that a part of the second windows 1120 is exposed and not covered by the respective hinge sheets 2000.

In the artificial heart valve prosthesis according to this embodiment, the hinge sheets 2000 can distribute stress within joints of the leaflets 3100 with the stent 1000 (i.e., around the second windows 1120), resulting in an extended service life of the leaflets 3100. Moreover, at least one of the hinge sheets 2000 and the flaps 3120 is sutured at the first windows 1110, effectively preventing the valve 3000 from sagging, when it closes, due to blood pressure and thereby improving its use. The present invention is not limited to any particular material of the hinge sheets 2000, and they may be made of either a polymer material or a natural biological material.

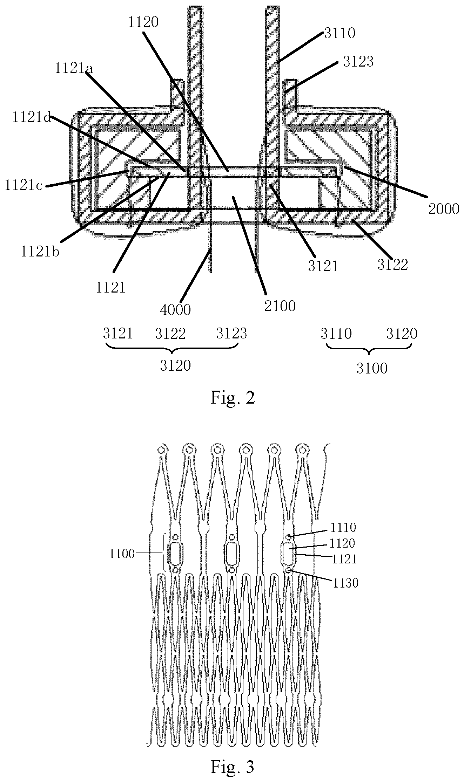

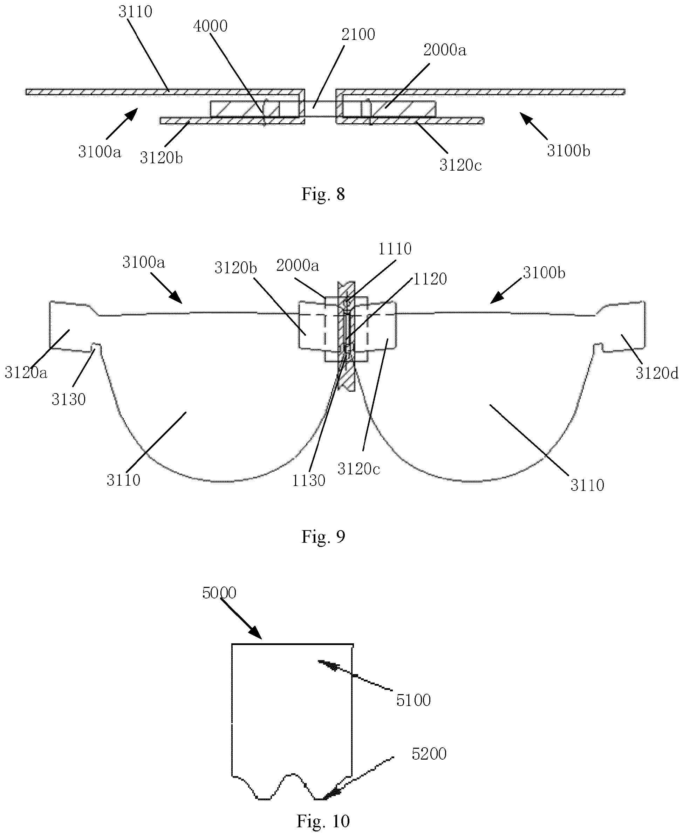

Optionally, with continued reference to Figs. 3 and 4, in each commissure 1100, a third window 1130 may be additionally provided on a side of the second window 1120 closer to the inflow end 1001. In this case, the hinge sheets 2000 may be sutured at the third windows 1130 (i.e., the hinge sheets 2000 may be sutured to the commissures 1100 by suture threads passed through the third windows 1130). This can enhance stress distribution at the joints of the leaflets 3100 with the stent 1000. In this embodiment, reference to Fig. 5, in conjunction with Fig. 9, the hinge sheets 2000 are substantially rectangular and have a dimension in the axial direction of the stent 1000 which is comparable to that of the commissures 1100 so as to be able to cover both the first windows 1110 and the third windows 1130. In alternative embodiments, the hinge sheets 2000 may cover one of the first windows 1110 and the third windows 1130. Compared with not covering the first windows 1110 and/or the third windows 1130 with the hinge sheets 2000, covering them with the hinge sheets 2000 enables more firm suturing of the hinge sheets 2000 to the first windows 1110 and/or the third windows 1130.

Referring back to Fig. 2, in conjunction with Fig. 3, side support arms 1121 that delimit the second windows 1120 are distributed circumferentially around the stent 1000. Each side support arm 1121 has, sequentially joined to one another end-to-end, an inner side face 1121a (facing toward an inner surface of the second window), an outer surface 1121b (facing toward an outer surface of the stent and forming a part of the outer surface of the respective commissure), an outer side face 1121c (facing away from a side surface of the second window) and an inner surface 1121d (facing toward an inner surface of the stent and forming a part of an inner surface of the respective commissure). At least a part of the outer surface 1121b of each side support arm 1121 is covered by the respective hinge sheet 2000. Preferably, the outer side face 1121c and the inner surface 1121d are also covered by the respective hinge sheet 2000.

Each flap 3120 includes a head portion 3121, a middle portion 3122 and a trailing portion 3123, which are sequentially joined together. The head portion 3121 is inserted in both the respective second window 1120 (i.e., the head portion 3121 covers the inner side face 1121a of the respective side support arm 1121) and the respective through hole 2100. The middle portion 3122 extends over the outer surface 1121b, the outer side face 1121c and the inner surface 1121d of the respective side support arm 1121 so as to surround the portion of the respective hinge sheet 2000 arranged on the respective side support arm 1121. The trailing portion 3123 overlaps a part of the main segment 3110 of the corresponding leaflet 3100. In this way, the portions of each hinge sheet 2000 arranged on the respective side support arms 1121 of the respective second window 1120 are completely wrapped by the respective leaflet 3100, facilitating uniform distribution of stress at the joints of the leaflets 3100 with the stent 1000 across the entire commissures 1100. This can avoid stress concentration and enhance durability of the stent 1000. In addition, since the hinge sheets 2000 cover the outer surfaces 1121b, the outer side faces 1121c and the inner surfaces 1121d of the side support arms, the side support arms 1121 are separated from the flaps 3120, avoiding friction from occurring between the side support arms 1121 and the flaps 3120, and extending the life of the leaflets 3100.

Further, since the trailing portions 3123 of the flaps 3120 overlap portions of the main bodies 3110, the flaps 3120 may be overseamed with suture threads 4000. As shown in Fig. 2, the overseam means that: the suture threads 4000 are passed successively through the main bodies 3110, the second windows 1120, the through holes 2100 and the trailing portions 3123 and then again through the main bodies 3110. With this seaming approach, the suture threads 4000 surround the side support arms 1121 to directly bind the stent 1000 and are prevented from coming into contact with the commissures 1100, eliminating the risk of breakage of the suture threads 4000 due to rubbing against the commissures 1100.

In order to enable the flaps 3120 to be neatly overseamed at the second windows 1120, the through holes 2100 in the hinge sheets 2000 are preferred to have a width greater than or equal to a width of the second windows 1120. Here, the widths of the through holes 2100 and the second windows 1120 refer to their extents in the circumferential direction of the stent 1000. As such, in the circumferential direction of the stent 1000, edges of the through holes 2100 are located laterally to the second windows 1120, or coincide with edges of the second windows 1120.

Those skilled in the art would appreciate that the artificial heart valve prosthesis has equal numbers of leaflets 3100, hinge sheets 2000 and commissures 1100, which may be determined as required by practical applications. For example, when the artificial heart valve prosthesis is used to replace a mitral valve, there may be two or more leaflets 3100, hinge sheets 2000 and commissures 1100. As another example, when the artificial heart valve prosthesis is used to replace a tricuspid valve, there may be three or more leaflets 3100, hinge sheets 2000 and commissures 1100. Generally, in the artificial heart valve prosthesis, the multiple commissures 1100 are arranged on the same circumference.

In the context of the artificial heart valve prosthesis including two leaflets 3100, two hinge sheets 2000 and two commissures 1100 in the stent 1000 as an example, assembly of the artificial heart valve prosthesis will be described below with reference to Figs. 2 and 7 to 9. In the following description, the terms "left", "right", "upper", "lower" and the like are meant to be used with respect to the configuration shown in Fig. 7 or 9. They are intended merely to facilitate and simplify the explanation of the invention and do not indicate or imply that the stated components or elements have to comprise, or be constructed or operated in, particular orientations. Therefore, they are not to be construed as limiting the invention.

In the following description, for ease of distinction, the two leaflets 3100 are referred to respectively as a first leaflet 3100a and a second leaflet 3100b, the two flaps 3120 of the first leaflet 3100a as a first left flap 3120a and a first right flap 3120b, the two flaps 3120 of the second leaflet 3100b as a second left flap 3120c and a second right flap 3120d, and the two hinge sheets 2000 as a first hinge sheet 2000a and a second hinge sheet (not shown).

At first, as shown in Fig. 7, the first right flap 3120b of the first leaflet 3100a is passed through the through hole 2100 of the first hinge sheet 2000a, and the second left flap 3120c of the second leaflet 3100b is passed through the through hole 2100 of the first hinge sheet 2000a.

Subsequently, the first right flap 3120b is bent to the left, and the first hinge sheet 2000 is sutured to the first right flap 3120b with a suture thread 4000. Similarly, the second left flap 3120c is bent to the right, and the first hinge sheet 2000a is sutured to the second left flap 3120c with a suture thread 4000 (see Fig. 8).

The above process is then repeated to hinge the first left flap 3120a of the first leaflet 3100a and the second right flap 3120d of the second leaflet 3100b to the second hinge sheet.

After that, the first hinge sheet 2000a, the first right flap 3120b and the second left flap 3120c are inserted, as a whole from the inner side of the stent 1000, through one of the second windows 1120 and then straighten the first hinge sheet 2000a, the first right flap 3120b and the second left flap 3120c (see Fig. 9). Moreover, the second hinge sheet, the first left flap and the second right flap are inserted, as a whole from the inner side of the stent 1000, through the other second window and then straighten the second hinge sheet, the first left flap and the second right flap (not shown).

Next, the hinge sheets 2000 and the flaps 3120 are bent in the manner as shown in Fig. 2 so that each hinge sheet 2000 covers the two side support arms 1121 of a corresponding second windows 1120 and that the flaps 3120 cover the respective hinge sheets 2000.

After that, upper portions of the hinge sheets 2000 are sutured to the respective first windows 1110 (not shown) with suture threads. Afterward, the flaps 3120 are sutured down to the respective second windows 1120 along the axial direction of the stent 1000 in the manner as described above. Finally, lower portions of the hinge sheets 2000 are sutured at the respective third windows 1130 (not shown).

Preferably, the flaps 3120 are provided with, on their side closer to the inflow end 1001 of the stent 1000, engagement notches 3130 at their junctions with the main bodies 3110, which are adapted for location during assembly. Specifically, during assembly of the leaflets 3100 with the hinge sheets 2000, the engagement notches 3130 may engage the through holes 2100 of the hinge sheets 2000. Moreover, during assembly of the leaflets 3100 and the hinge sheets 2000 with the stent 1000, the engagement notches 3130 may engage lower edges of the second windows 1120.

Further, the main segment 3110 of each leaflet 3100 has a fixed edge 3111 and a free edge 3112. The fixed edge 3111 is disposed closer to the inflow end 1001 of the stent 1000 and is attached to the stent 1000. The free edge 3112 is disposed closer to the outflow end 1002 of the stent 1000. When the valve 3000 opens, the free edges 3112 of all the leaflets 3100 separate from one another. When the valve 3000 closes, the free edges 3112 of all the leaflets 3100 fit together. Preferably, in the axial direction of the stent 1000, a distance from the free edges 3112 to the outflow end 1002 of the stent 1000 is greater than a minimum distance from the flaps 3120 of the leaflets 3100 to the outflow end 1002. With this arrangement, in the closed configuration of the valve 3000, the leaflets 3100 are pulled at locations lower than locations where the stent 1000 is pulled. This can reduce stress on the stent 1000 and improve durability of the stent 1000.

Preferably, each fixed edge 3111 is substantially a V-shaped structure with a rounded corner. This shape of the fixed edges 3111 can reduce stress on the leaflets 3100 and improve durability of the valve 3000. Moreover, it can mitigate creasing and wrinkling of central portions of the leaflets 3100. This can reduce early calcification and the amount of material used.

Further, as shown in Figs. 1 and 10, the artificial heart valve prosthesis further includes a skirt 5000 including at least an inner skirt 5100 disposed on an inner side of the inflow end 1001 of the stent 1000. The inner skirt 5100 may be connected to the fixed edges 3111 of the main bodies 3110. The skirt 5000 may further include an outer skirt 5200 disposed on an outer side of the inflow end 1001 of the stent 1000. The outer skirt 5200 may be integral with the inner skirt 5100. An edge of the outer skirt 5200 away from the inner skirt may be wavy in shape in order to reduce the amount of material used.

It is to be noted that in the foregoing embodiments, the hinge sheets 2000 are not mandatory. In alternative implementations, they may be omitted. In these cases, the flaps 3120 of the leaflets 3100 may be brought into direct contact with the commissures 1100 of the stent 1000.

Embodiments of the present invention also provide another artificial heart valve prosthesis, which is substantially the same as the artificial heart valve prosthesis according to the above embodiments, except that no first windows are formed in the stent. In other words, the artificial heart valve prosthesis according to these embodiments includes: a stent comprising at least two commissures, which are spaced circumferentially around the stent, each of the commissures comprising a second window; hinge sheets, each covering at least a part of an outer surface of a respective one of the commissures and comprising a through hole; and at least two leaflets disposed circumferentially around the stent, each of the leaflets including a main segment and flaps arranged on opposite circumferential sides of the main segment, the main segment disposed on an inner side of the stent, each of the flaps passed through the second window of a respective one of the commissures and the through hole of a respective one of the hinge sheets and covering at least a part of an outer surface of the respective hinge sheet, the flaps are sutured at the second window.

It would be appreciated that, as each hinge sheet covers at least a part of the outer surface of the respective commissure and each flap covers at least a part of the outer surface of the respective hinge sheet, the hinge sheets are at least partially situated between the commissures and the flaps. In this way, when suturing the flaps at the second windows, the hinge sheets are sandwiched and secured between the flaps and the commissures.

For other structural details of this artificial heart valve prosthesis, reference can be made to the description of the preceding embodiments, and further description thereof is therefore omitted here. Although the present invention has been disclosed hereinabove, it is not limited to the above disclosure. Those skilled in the art can make various changes and modifications to the invention without departing from the spirit and scope thereof. Accordingly, it is intended that any and all such changes and modifications also fall within the scope of the present invention as defined by the appended claims and equivalents thereof.

## Claims

1. An artificial heart valve prosthesis, comprising:
a stent comprising at least two commissures spaced circumferentially around the stent, wherein each of the commissures is provided with a first window and a second window, which are arranged along an axial direction of the stent, and wherein the first window is located closer to an outflow end of the stent; and
at least two leaflets arranged circumferentially around the stent, wherein each of the leaflets comprises a main segment and flaps arranged on opposite circumferential sides of the main segment, wherein the main segment is disposed on an inner side of the stent, each of the flaps passed through the second window and at least partially covering an outer surface of the commissure, and wherein the flap is sutured at the first window and the second window.

2. The artificial heart valve prosthesis according to claim 1, further comprising hinge sheets, wherein each of the hinge sheet is at least partially arranged between the commissure and the flap of the leaflet, wherein the hinge sheet is provided with a through hole for passage therethrough of the flap, and wherein the hinge sheet is sutured at the first window.

3. The artificial heart valve prosthesis according to claim 2, wherein the hinge sheet covers the first window, or wherein the flap is sutured to the hinge sheet.

4. The artificial heart valve prosthesis according to claim 2, wherein the second window is delimited by side support arms arranged circumferentially around the stent, wherein:
the hinge sheet at least partially covers an outer surface, an outer side face and an inner surface of the side support arm; and
each of the flaps comprises a head portion, a middle portion and a trailing portion, which are sequentially joined, wherein the head portion is inserted in the second window and the through hole, wherein the middle portion extends over the outer surface, the outer side face and the inner surface of the side support arm so as to wrap a portion of the hinge sheet arranged on the side support arm, and wherein the trailing portion overlaps a portion of the main segment in the leaflet.

5. The artificial heart valve prosthesis according to claim 4, wherein the flap is sutured at the second window by a suture thread successively passed through the main segment of the leaflet, the second window, the through hole and the trailing portion of the flap.

6. The artificial heart valve prosthesis according to any one of claims 2 to 5, wherein in a circumferential direction of the stent, edges of the through hole are coincident with or lateral to edge of the second window.

7. The artificial heart valve prosthesis according to any one of claims 2 to 5, wherein that the flap is provided with an engagement notch on a side thereof proximate an inflow end of the stent, wherein the engagement notch is located at a junction of the flap with the main segment and is configured to engage the through hole and the second window.

8. The artificial heart valve prosthesis according to any one of claims 2 to 5, wherein the commissure is further provided with a third window, wherein the third window is located on a side of the second window proximate an inflow end of the stent; and
wherein the hinge sheet is sutured at the third window.

9. The artificial heart valve prosthesis according to claim 1, wherein the second window is delimited by side support arms arranged circumferentially around the stent,
wherein each of the flaps comprises a head portion, a middle portion and a trailing portion, which are sequentially joined, wherein the head portion is inserted in the through hole, wherein the middle portion extends over an outer surface, an outer side face and an inner surface of the side support arm, and wherein the trailing portion overlaps a portion of the main segment in the leaflet.

10. The artificial heart valve prosthesis according to claim 9, wherein the flap is sutured at the second window by a suture thread successively passed through the main segment of the leaflet, the second window and the trailing portion of the flap.

11. An artificial heart valve prosthesis, comprising:
a stent comprising at least two commissures spaced circumferentially around the stent, wherein the commissure is provided with a second window;
hinge sheets at least partially covering an outer surface of the commissure and provided with a through hole; and
at least two leaflets arranged circumferentially around the stent, wherein each of the leaflets comprises a main segment and flaps arranged on opposite circumferential sides of the main segment, wherein the main segment is disposed on an inner side of the stent, wherein the flap is passed through the second window and the through hole, and covers at least a part of an outer surface of the hinge sheet, and wherein the flap is sutured at the second window.

12. The artificial heart valve prosthesis according to claim 11, wherein the second window is delimited by side support arms arranged circumferentially around the stent, wherein:
the hinge sheet at least partially covers an outer surface, an outer side face and an inner surfaces of the side support arm; and
each of the flaps comprises a head portion, a middle portion and a trailing portion, which are sequentially joined, wherein the head portion is inserted in the second window and the through hole, wherein the middle portion extends over the outer surface, the outer side face and the inner surface of the side support arm so as to wrap a portion of the hinge sheet arranged on the side support arm, and wherein the trailing portion overlaps a portion of the main segment in the leaflet.

13. The artificial heart valve prosthesis according to claim 12, wherein the flap is sutured at the second window by a suture thread successively passed through the main segment of the leaflet, the second window, the through hole and the trailing portion of the flap.

14. The artificial heart valve prosthesis according to any one of claims 11 to 13, wherein in a circumferential direction of the stent, edges of the through hole is coincident with or lateral to edges of the second window.

15. The artificial heart valve prosthesis according to any one of claims 11 to 13, wherein the flap is provided with an engagement notch on a side thereof proximate an inflow end of the stent, wherein the engagement notch is located at a junction of the flap with the main segment and is configured to engage the through hole and the second window.

16. The artificial heart valve prosthesis according to any one of claims 11 to 13, wherein the commissure is further provided with a third window, wherein the third window is located on a side of the second window proximate an inflow end of the stent,
wherein the hinge sheet is sutured at the third window.
